# EUROPEAN PATENT SPECIFICATION

(11) **EP 0 400 548 B1**
(45) Date of publication and mention of the grant of the patent: **16.08.1995**
(21) Application number: 90110106.3
(22) Date of filing: 28.05.1990
(51) Int. Cl.: G01N 33/569, A61K 39/21, G01N 33/96

(54) **Standard reagents for the quantitation of HIV p24**
Standard-Reagenzien zur Quantifizierung von HIV p24
Réactifs de standard pour la quantification de HIV p24

(30) Priority: 02.06.1989 US 360661
(43) Date of publication of application: 05.12.1990
(73) Proprietor: ABBOTT LABORATORIES, Abbott Park, Illinois 60064-3500 (US)
(72) Inventor: Stewart, James Lawrence, Gurnee, IL 60031 (US); Mc Straw, Regina H., Gurnee, IL 60031 (US)
(74) Representative: Modiano, Guido, Dr.-Ing.

(56) References cited:
- EP-A- 0 007 163
- EP-A- 0 216 191
- WO-A-79/00106

## Description

### BACKGROUND OF THE INVENTION

Enzyme immunoassays have been utilized for the detection of antibody to HIV in a biological sample. Although such tests are highly sensitive indicators of past exposure to HIV, they give no direct evidence for the presence of the virus. Detection of viable virus in blood and other body fluids can often be achieved only through complex culture procedures (Salahuddin et al., *PNAS* (1985) 82:5530-5534). U.S. Patent No. 4,748,110 describes an enzyme immunoassay for the detection of HIV antigens.

Determining varying levels of viral antigen in HIV infected individuals has been useful in monitoring patient response to antiviral therapies. One semi-quantitative method which has been widely used for this purpose involves obtaining a standard curve by assaying, in parallel with samples being tested for antigen, serial dilutions of a positive control reagent, which is made up of viral lysate antigens. Intensities of signals from immunoassays (e.g. absorbances from ELISAs, in which the enzyme catalyzes production of a chromogenic product) of patient samples can then be converted to antigen concentrations by simply locating the intensities on the standard curve. This method is only semi-quantitative because its accuracy is severely limited by the lack of purity and consistency of the viral lysate antigen preparations used as the positive control reagent, the instability of these mixtures of antigens in solution, and the difficulty of having sufficient reproducibility in the serial dilutions.

It is known that the concentration of the HIV protein known as "p24" in the blood serum or plasma of persons infected with HIV varies with the severity of immune suppression in such persons. Thus, there is a need for a quantitatively accurate assay for HIV p24 to monitor disease progression and the effectiveness of therapeutic methods in persons infected with HIV.

### SUMMARY OF THE INVENTION

The present invention is directed to improved immunoassay methods, and compositions and kits for carrying out the methods, for quantitating (i.e., determining the titer of) HIV p24 antigen in a biological sample.

It has been discovered that it is especially advantageous to employ purified viral HIV p24 as the antigenic reagent in control samples employed to establish the relationship between titer of HIV p24 in a sample and the intensity of signal from an immunoassay of the sample for HIV p24. Thus, the methods of the invention make possible the quantitation of HIV p24 with accuracy that heretofore has been unattainable.

### DETAILED DESCRIPTION OF THE INVENTION

In one of its aspects, the present invention is a method for quantitating HIV p24 in a biological sample, comprising the steps of:
contacting said sample with an antibody specific for HIV p24 antigen, whereby an antibody-antigen complex is formed; and
quantitating the amount of antigen-antibody complex formed employing as a standard a quantitation panel which comprises compositions comprising purified viral HIV p24.

This method of the invention is a significant improvement over prior art immunoassay methods for detecting HIV viral antigens in biological samples. The improvement lies in the use of purified viral HIV p24 in the quantitation panel.

In another of its aspects, the invention entails a composition suitable as a HIV p24 standard, which composition comprises purified viral HIV p24 and a buffer.

In still a further aspect, the invention entails a kit for use in quantitating HIV p24 in a biological sample, said kit comprising a quantitation panel which comprises compositions comprising purified viral HIV p24.

By "purified viral HIV p24" is meant the HIV p24, obtained from HIV-1, HIV-2 or other strains of the virus, or HIV p24 prepared by genetic engineering techniques that is immunologically indistinguishable from the p24 obtained from a strain of the virus, provided that the p24 constitutes at least 95% of the protein in the sample which includes the p24. Purified viral HIV p24 can be prepared as described below employing immunoaffinity purification from a HIV viral antigen preparation using a monoclonal antibody against the p24.

Reference herein to "an antibody specific to HIV p24 antigen" in a method according to the invention means either an antibody that recognizes an epitope on viral HIV p24 but is part of a polyclonal antibody preparation or a monoclonal antibody that recognizes an epitope on viral HIV p24.

The antigen-antibody complex formed in a method according to the invention can be detected by any of the numerous methods known in the immunoassay art for detecting such complexes. For example, the antibody specific to HIV p24 may be labeled directly with a detectable label or a moiety, such as an enzyme, capable of generating a detectable signal, or ELISA methods or other sandwich assay methods employing a radiolabeled second antibody can be employed. Alternatively, competitive assay methods can be employed, wherein HIV p24 of a sample competes with labeled, purified viral HIV p24 for binding to an antibody specific for HIV p24.

A "quantitation panel" in accordance with the invention can be a set of solutions or a set of quantities of dry (e.g., lyophilized) reagents. A quantitation panel that is a set of quantities of dry reagents can be converted to quantitation panel that is a set of solutions merely by adding water or an appropriate salt solution or aqueous solution of buffer(s) and salt(s) as well understood in the art and illustrated infra. While a preferred buffer is Tris, other buffers such as HEPES (N-[2-Hydroxyethyl]piperazine-N'-[2-ethanesulfonic acid]), PIPES (Piperazine-N,N'-bis[2-ethanesulfonic acid]), MOPS (3-[N-Morpholino]propanesulfonic acid), BES (N,N-bis[2-Hydroxyethyl]-2-aminoethanesulfonic acid), TES (N-tris[Hydroxymethyl]methyl-2-aminoethanesulfonic acid) and Phosphate (monobasic and dibasic) may be employed, as understood in the art. While NaCl is a suitable salt in the quantitation panels of the invention, other salts such as KCl can also be employed, also as understood in the art.

As those of skill in the art will readily understand, a "control composition" in a quantitation panel according to the invention will be a composition that lacks HIV p24 but is otherwise the same in composition as the compositions which include HIV p24 and are part of the quantitation panel of which the control composition is also a part.

In accordance with the invention, the compositions of a quantitation panel are used to establish a relationship (e.g., a "standard curve") between intensity of signal due to antigenantibody complex formed in accordance with the method of the invention and concentration of HIV p24 in a sample. Thus, a quantitation panel, in which the compositions are solutions, will have compositions with at least two different concentrations of HIV p24, and usually more than 3 different concentrations with one which is 0 (i.e., lacks p24). The range of concentrations in a quantitation panel, in which the compositions are solutions, will be from 0 to about 400 pg of purified viral HIV p24 per ml of solution, more preferably from 0 to about 300 pg of said p24 per ml, and still more preferably from about 0 to about 100 of said p24 per ml. In the compositions of a panel, which are solutions and which comprise purified viral HIV p24, the range of concentrations of the p24 will be from about 0.5 pg/ml to about 400 pg/ml, more preferably from about 1 pg/ml to 300 pg/ml and still more preferable from about 10 pg/ml to about 100 pg/ml.

The sensitivity and reproducibility of quantitating HIV p24 in HIV antigen-positive samples in accordance with the method of the present invention were assessed in comparison with the sensitivity and reproducibility of results obtained with the current, semi-quantitative procedure, wherein a mixture of antigens from a viral lysate is employed to establish a standard curve. The present method allowed quantitation of p24 itself to less than 1 pg/ml. The quantitation panel of the invention, wherein components of the compositions were lyophilized, provided assays with superior reproducibility (typical intra-assay variation, <10%, interassay, <15%).

The improvement in purity, by the use of purified HIV p24, enhances stability of quantitation panels and the reproducibility of assays carried out therewith and provides, therefore, a significant improvement over the assays employing viral lysate antigens for standards in the reliability of measuring patient antigen levels.

The use of monoclonal antibodies in affinity purification produced viral p24 antigen of at least 95% purity, which is utilized in the panel. Lyophilization gave stability to the panel for over 8 months at 2-8°C and the buffer enhanced stability of the reconstituted panel at 2-8°C for more than 6 weeks. The reproducibility of the present method of quantitative values from serum and plasma samples has shown less than 15% variation compared to 40% in the current semi-quantitative procedure. The method of the invention for quantitating HIV p24 in a biological sample, which utilizes purified viral p24, because of its improved purity, improved stability of the standards and reproducibility of the quantitative values obtained from the standard curve, is a useful and reliable tool for monitoring p24 antigen levels in patients on antiviral therapies.

Biological samples which are easily tested by the method of the present invention include human and animal body fluids and tissues such as plasma and serum, as well as tissue culture media.

The buffer used in the solution to dilute the purified viral HIV p24 lends better stability to the panel after reconstitution. Preferably, the solution is a buffered saline solution. For example, Tris(Tris[hydroxymethyl]aminomethane), commercially available from Sigma Chemical Co., St. Louis, MO, can be used to buffer the saline.

The p24 used in the present invention can be obtained from HIV-1 infected cells. A variety of HIV-1 isolates have been identified, among which is the isolate produced by HTLV-III(b)-infected H9 cells, which are available to the skilled in the art. (HTLV-III isolates are now designated HIV-1). Thus, any cell which harbors an HIV-1 virus will be an appropriate source for the p24 antigens.

One of ordinary skill in the art can utilize the methods disclosed herein to develop a quantitation standard for any other human immunodeficiency virus, such as HIV-2, by obtaining purified viral p24 therefrom.

### METHODS

### Protein p24 Purification

H9 cells infected with HTLV-III(b) were harvested at their peak log phase of growth (approximately 7-10 days). The cells were concentrated by ultrafiltration and then pelleted by centrifuging at 20,000 RPM overnight (or, alternatively, 25,000 RPM for 3 hours or 43,000 PPM for 2 hours). The pelleted cells were resuspended in a solution containing 1 mM Tris, 15 mM NaCl and 0.1 mM EDTA, pH 7.5 (TNE).

The resuspended pellet was clarified by centrifuging at 10,000 RPM for approximately 15 minutes. The supernatant was decanted, and the pellet was resuspended in TNE and centrifuged again. The supernatants were combined and centrifuged one more time.

Virus contained in the supernatant obtained above was pelleted through a 20% sucrose pad by layering a 20% sucrose solution under the supernatant in a centrifuge tube and centrifuging at 35,000 RPM for 4 hours at 2-8°C (or, alternatively, 20,000 RPM for 16-22 hours or 43,000 RPM for 2 hours 45 minutes). The pellets were resuspended in TNE.

The resuspended virus was then banded by equilibrium sedimentation in a sucrose gradient according to procedures well known to those skilled in the art (see for example, the method of Sundquist, *Archives of Virology* (1981) 68:115-127). Briefly, the resuspended virus was layered on top of a sucrose gradient prepared as follows. In a centrifuge tube, the gradient was prepared by underlaying a 45% sucrose solution to a 30% sucrose solution, overlaying the resuspended virus on the gradient and centrifuging at 25,000 RPM overnight in a swinging bucket rotor (e.g., SW-25). The gradient was fractionated and those fractions containing protein showing p41 activity were pooled. The banded virus was diluted in TNE and pelleted by centrifuging at 35,000 RPM for 4 hours at 2-8°C (or, alternatively, 20,000 RPM for 16-22 hours or 43,000 RPM for 2 hours 45 minutes). The pelleted virus was resuspended in TNE, sonicated in a solution containing 0.5 M NaCl and 0.5% Triton® X-100, and clarified by centrifuging at 35,0000 RPM for 4 hours (or 43,000 RPM for 2 hours 40 minutes). The supernatant was decanted to provide purified viral lysate.

The p24 protein was isolated from the purified viral lysate by immunoaffinity chromatography using a monoclonal antibody. (General methods of monoclonal antibody production are reviewed in *Monoclonal Hybridoma Antibodies: Techniques and Applications,* ed. J.G.R. Hurrell [CRC Press, Inc.,1982]). Briefly, sepharose gel, for example Sepharose® 4B (Pharmacia Fine Chemicals Inc., Uppsala, Sweden), was first coupled with a monoclonal antibody specific for HIV-1 p24, cross-linked with glutaraldehyde at 0.05% and washed with a solution containing 0.01 M Tris, 0.001 M EDTA, 0.75 M NaCl, and 0.5% Triton® X-100, pH 8.1 (wash solution). The washed gel was then mixed with the purified viral lysate (2 mg antigen/ml gel) and tumbled on a rotator for 12-24 hours at 2-8°C. The lysate-gel mixture was then poured into a column, washed with 5 column volumes of wash solution, and the p24 was eluted with 4.0 M guanidine HCL. The fractions containing protein were pooled, and the eluted p24 dialyzed overnight in a 0.017 M phosphate solution containing 0.145 M NaCl, pH-7.2.

A protein assay, for example using Bio-Rad reagents (Bio-Rad Laboratories, Richmond, CA) according to the manufacturer's procedures, was conducted to determine protein concentration, and corrected for absolute p24 protein concentration by the use of scanning densitometry. By these procedures, preparations of antigen with greater than 95% HIV p24 were routinely made.

### Panel Members Preparation

The quantitation panel consists of a five member panel with concentrations of 100, 50, 25, 12.5 and 0 pg/ml p24. The panel is made by diluting the purified p24 antigen stock in an aqueous solution of buffer and NaCl to a final concentration of 100 pg/ml. The other panel members can be prepared by serial dilution of the first member. Alternatively, each individual panel member can be prepared by diluting the purified p24 antigen stock in the aqueous solution of buffer and NaCl to the desired final concentration.

Quantitation panel lot one (QP1) was prepared as follows. The 100 pg(ml panel member was prepared by diluting a stock solution of purified HIV p24 antigen of known concentration (17.0 ng/ml) with 0.02 M Tris, pH 7.2, containing 150 mM NaCl, 1% bovine serum albumin (BSA) and 0.1% NaN₃ (0.02 M Tris). Fifteen hundred milliliters of 0.02 M Tris were mixed with 8.82 ml of the HIV p24 antigen stock solution. Upon testing, the concentration was low; an additional 0.59 ml of the stock was added. The other members were prepared by serially diluting the 100 pg/ml member.

### Lyophilization Procedures

All lyophilization cycles are defined in terms of soak and ramp segments. A soak segment is defined as maintaining a set point value for a specific interval of time. A ramp segment is defined as the rate or time at which the set point changes. The basic cycle configuration has three segments: (1) an initial shelf/product cool segment (soak); (2) a shelf/product heating segment during which chamber pressure is controlled (ramp); and (3) a final shelf/product drying segment, still controlling chamber pressure (soak).

A Hull Lyophilizer was used, following general instructions by the manufacturer (Hull Corporation, Hatboro, Pennsylvania). The lyophilization cycle was based on product pre-cool requirements, product freezing temperature, absolute chamber pressure during the drying cycle and the desired shelf drying temperature. The lyophilizer shelves were pre-cooled to -45°C or lower. The filled vials were placed on the shelves, and the product was allowed to freeze until the temperature reached -20°C and remained at that temperature or lower for at least 3 hours. The drying cycle was then started by cooling the condenser plates and pulling a vacuum. The setpoint for the vacuum was 100 »m (microns). The shelf temperature was ramped from a setpoint of -40°C to 25°C over an 8.0 hour period. The shelf temperature was held at 25°C, and the product was allowed to reach a constant temperature above 0°C and remained at that temperature from 7 to 20 hours, prior to stoppering. The vials were stoppered under 17 to 51 kPa (5 to 15 inches of mercury) vacuum using nitrogen to fill the chamber.

Two other quantitation panel lots (QP2, QP3) were prepared as described above.

### HIV Antigen Assay Testing Procedures

An enzyme immunoassay for the detection of HIV-1 antigens (Ag) was used to quantitate p24, commercially available from Abbott Laboratories, Abbott Park, IL. Briefly, polystyrene beads coated with human antibody to HIV-1 were incubated with either a specimen or member of the p24 quantitation panel at room temperature for 16 to 20 hours. After incubation, unbound materials were aspirated and the beads were washed. Rabbit antibody was then incubated with the bead at 40°C for 4 hours, which bound to the HIV-1 Ag. Unbound materials were aspirated and the beads were washed. Goat antibody to rabbit IgG conjugated with horseradish peroxidase was incubated with the bead at 40°C for 2 hours, which bound to the rabbit antibody. Unbound materials were aspirated and the beads were washed. Next, o-Phenylenediamine (OPD) solution containing hydrogen peroxide was added to the bead, and, after incubation for 30 minutes, a yellow-orange color developed, in proportion to the amount of HIV-1 Ag bound to the bead. The enzyme reaction was stopped by the addition of 1 N H₂SO₄. The intensity of color was read using a spectrophotometer at 492 nm.

The presence or absence of HIV-1 Ag is determined by relating the absorbance of the specimen to a cutoff value. The cutoff value is the absorbance of the negative control mean or cell culture mean plus the factor 0.050. Specimens with absorbance values greater than or equal to the cutoff value are considered reactive for HIV-1 Ag. Specimens with absorbance values less than the cutoff value are considered nonreactive for HIV-1 Ag.

### EXAMPLES

### Example 1

### Assay Reproducibility

The five member panel was tested in the HIV antigen assay according to the protocol outlined above. The absorbance values of the panel members were plotted versus their concentration values. The curves generated were substantially linear and very reproducible. With a particular antigen assay kit, tested at three different clinical sites, the combined mean correlation coefficient for QP1, QP2 and QP3 was 0.9967 (n=22) with a coefficient of variation (CV, standard deviation/mean) of 0.2%. Furthermore, good correlation was seen between different antigen assay kits for each quantitation panel.

Intra and inter-assay reproducibility of each quantitation panel member, as well as lot-to-lot reproducibility of the panel is very good. With a particular antigen assay kit, tested with all three quantitation panel lots at one clinical site, the absorbance value for each panel member is very reproducible. As shown in Table 1, the CV of each panel member was less than 10%.

**Table 1**

| Panel Member | Mean O.D. Value^{a} | %CV |
|---|---|---|
| A | 0.047 | 7.7 |
| B | 0.154 | 4.9 |
| C | 0.265 | 4.6 |
| D | 0.455 | 5.2 |
| E | 0.799 | 6.0 |

| | | |
|---|---|---|
| ^{a}Number of tests equals 28 | | |

### Example 2

### Sample Quantitation

A ten member sample set was quantitated against each of the three HIV p24 antigen quantitation panels, and %CVs were calculated. The sample set consisted of the following:
- Samples 1-3: purified p24 antigen spiked into normal human plasma at known concentrations (pg/ml);
- Sample 4: 1:2 dilution of plasma from an antigen positive donor (pg/ml);
- Samples 5-7: undiluted antigen positive donor plasma samples (pg/ml);
- Sample 8: purified p24 antigen spiked into RPMI 1640 tissue culture medium with 5% fetal bovine serum and 0.5% Triton® X-100 (pg/ml);
- Sample 9: purified p24 antigen spiked into tissue culture medium, as described above, but at a higher concentration which required a 1:100 dilution to be made before testing; and
- Sample 10: purified p24 antigen spiked into the aforementioned tissue culture medium, requiring a 1:300 dilution to be made before testing.

As shown below in Table 2, lot-to-lot reproducibility of the HIV p24 antigen panel across the three lots (QP1, QP2 and QP3) was excellent. The present method for quantitating HIV p24 in a biological sample gave assay values for plasma with an average variance of less than 7%, ranging from 4.5% to 11.2%. In addition, tissue culture samples which vary more because of the detergent content (0.5% Triton® X-100) and the dilution made before testing, showed less than 20% variance in the method of the invention.

**Table 2**

| Panel Member | Mean Assay Value | %CV |
|---|---|---|
| 1 | 22.9 | 4.5 |
| 2 | 86.2 | 6.3 |
| 3 | 130.9 | 6.8 |
| 4 | 26.5 | 6.4 |
| 5 | 39.8 | 5.7 |
| 6 | 49.6 | 5.3 |
| 7 | 39.5 | 11.2 |
| 8 | 52.8 | 8.4 |
| 9 | 17.6 | 7.2 |
| 10 | 37.2 | 17.6 |

In addition, the sample set was quantitated using each of the three HIV p24 antigen quantitation panels (QP1 , QP2 and QP3) against three different HIV antigen assay kits. For each quantitation panel, interassay reproducibility across the three different HIV Antigen assay kits was very good. As illustrated below in Table 3, reproducibility of the HIV antigen panel QP1 was excellent. The method of the invention for quantitating HIV p24 in a biological sample gave assay values for plasma with an average variance of less than 7%, ranging from 4.8% to 10.8%. In addition, tissue culture samples, containing 0.5% Triton® X-100 showed less than 20% variance.

**Table 3**

| Panel Member | Mean Assay Value (QP1) | %CV |
|---|---|---|
| 1 | 21.4 | 4.8 |
| 2 | 80.8 | 5.0 |
| 3 | 121.8 | 6.2 |
| 4 | 24.8 | 6.4 |
| 5 | 36.3 | 7.6 |
| 6 | 46.2 | 6.3 |
| 7 | 35.5 | 10.8 |
| 8 | 51.0 | 7.8 |
| 9 | 17.1 | 10.2 |
| 10 | 36.2 | 18.8 |

Moreover, in a sampling of 100 plasma and serum clinical samples tested for HIV antigen, 91% of those samples were able to be quantitated within the limits of the curve, i.e., their concentration was between 0 and 100 pg/ml p24.

### Example 3

### Comparative Data

A current quantitation procedure consists of serially diluting a positive control reagent provided in a diagnostic kit for the detection of HIV-1 antigens to provide a standard curve. The disadvantage of this method is the amount of p24 is indirectly measured because the positive control is a viral lysate solution. The viral lysate solution is derived from tissue culture and contains many kinds of proteins. The disclosed method of the invention significantly improves the reproducibility of positive samples as compared to the current procedure utilizing positive control dilutions. The data shown below in Table 4 summarize the variances of positive samples for both the current procedure and the procedure of the invention. Each positive sample was tested in both procedures against five different HIV antigen assay kits. The present method improved the variance for each sample tested.

**Table 4**

| Sample (Dilution) | Current Procedure (%CV) | Quantitation Panel (%CV) | Δ%CV |
|---|---|---|---|
| 1a (1:20) | 20 | 13 | 35 |
| 1b (1:60) | 39 | 28 | 28 |
| 2 | 38 | 26 | 32 |
| 3 | 18 | 7 | 61 |
| 4 | 14 | 9 | 36 |
| 5 | 16 | 5 | 69 |
| 6 | 14 | 7 | 50 |

In summary, the %CV was improved 28-69% with the HIV p24 quantitation panel compared to the positive control dilutions, indicating the method of the present invention for quantitating HIV p24 in a biological sample is an improvement over the current procedure for measuring HIV p24 antigen levels.

### Example 4

### Quantitation Panel Linearity

The linearity of the quantitation panel extends beyond the 100 pg/ml limit. An experiment was conducted, wherein panel members were added to include the concentrations of 125, 150, 175 and 200 pg/ml. The curve thus generated was substantially linear, with a correlation coefficient of 0.9985.

## Claims (Claims for the following Contracting State(s): AT, BE, CH, DE, FR, GB, IT, LI, LU, NL)

1. A method for quantitating HIV p24 in a biological sample, comprising the steps of:
contacting said sample with an antibody specific for HIV p24 antigen, whereby a detectable antigen-antibody complex is formed; and
quantitating the amount of antigen-antibody complex formed employing as a standard a quantitation panel which comprises compositions comprising purified viral HIV p24.

2. The method according to Claim 1 wherein, in the quantitation panel, the compositions comprising purified viral HIV p24 are solutions comprising said purified viral HIV p24 and a buffer, and the concentrations of said purified viral HIV p24 in said solutions vary from 1 to 300 pg/ml and the quantitation panel further comprises a control composition which is a solution which comprises said buffer and lacks said purified HIV p24.

3. A composition suitable as a HIV p24 standard for use in the method according to Claim 1 comprising purified viral HIV p24 and a buffer.

4. The composition of Claim 3 in lyophilized form.

5. The composition of Claim 3, wherein said buffer is Tris and the composition further comprises NaCl.

6. The composition of Claim 4, wherein said buffer is Tris and the composition further comprises NaCl.

7. A kit for use in quantitating HIV p24 in a biological sample according to the method of Claim 1, comprising:
a quantitation panel which comprises compositions comprising purified viral HIV p24.

8. The kit of Claim 7 wherein, in the quantitation panel, the compositions comprising purified viral HIV p24 also comprise a buffer, and the quantitation panel further comprises a control composition which comprises said buffer and lacks said purified HIV p24.

9. A kit of Claim 8 wherein the compositions of the quantitation panel are lyophilized.

10. A kit of Claim 9 wherein, in the compositions of the quantitation panel, said buffer is Tris and the compositions further comprise NaCl.

## Claims (Claims for the following Contracting State(s): ES)

1. A method for quantitating HIV p24 in a biological sample, comprising the steps of:
contacting said sample with an antibody specific for HIV p24 antigen, whereby a detectable antigen-antibody complex is formed; and
quantitating the amount of antigen-antibody complex formed employing as a standard a quantitation panel which comprises compositions comprising purified viral HIV p24.

2. The method according to Claim 1 wherein, in the quantitation panel, the compositions comprising purified viral HIV p24 are solutions comprising said purified viral HIV p24 and a buffer, and the concentrations of said purified viral HIV p24 in said solutions vary from 1 to 300 pg/ml and the quantitation panel further comprises a control composition which is a solution which comprises said buffer and lacks said purified HIV p24.

3. A method according to Claim 1 wherein said compositions comprising purified viral HIV p24 further comprise a buffer.

4. A method according to Claim 3 wherein said compositions comprising purified viral HIV p24 and a buffer are in lyophilized form.

5. A method according to Claim 3 or 4 wherein said buffer is Tris and said compositions comprising purified viral HIV p24 and Tris further comprises NaCl.

## Patentansprüche (Patentansprüche für folgende(n) Vertragsstaat(en): AT, BE, CH, DE, FR, GB, IT, LI, LU, NL)

1. Verfahren zur quantitativen Bestimmung von HIV p24 in einer biologischen Probe, das folgende Schritte umfaßt:
In-Kontakt-Bringen der Probe mit einem Antikörper, der für das HIV p24 Antigen spezifisch ist, wodurch ein nachweisbarer Antigen-Antikörper-Komplex gebildet wird;
und quantitative Bestimmung der Menge an Antigen-Antikörper-Komplex, der gebildet wurde, wobei als Standard eine Mengenbestimmungstabelle verwendet wird, die Zusammensetzungen umfaßt, die gereinigtes virales HIV p24 enthalten.

2. Verfahren nach Anspruch 1, wobei in der Mengenbestimmungstabelle die Zusammensetzungen, die gereinigtes virales HIV p 24 enthalten, Lösungen sind, die das gereinigte virale HIV p 24 und einen Puffer enthalten, und wobei die Konzentrationen des gereinigten viralen HIV p24 in den Lösungen von 1 bis 300 pg/ml variieren, und wobei die Mengenbestimmungstabelle darüberhinaus eine Blindzusammensetzung umfaßt, die eine Lösung ist, welche den Puffer enthält und in der das gereinigte HIV p 24 fehlt.

3. Zusammensetzung, die zur Verwendung als Standard für HIV p 24 in dem Verfahren nach Anspruch 1 geeignet ist, die gereinigtes virales HIV p 24 und einen Puffer enthält.

4. Zusammensetzung nach Anspruch 3 in gefriergetrockneter Form.

5. Zusammensetzung nach Anspruch 3, wobei der Puffer Tris ist, und wobei die Zusammensetzung darüberhinaus NaCl enthält.

6. Zusammensetzung nach Anspruch 4, wobei der Puffer Tris ist, und wobei die Zusammensetzung darüberhinaus NaCl enthält.

7. Kit zur Verwendung bei der quantitativen Bestimmung von HIV p24 in einer biologischen Probe in dem Verfahren nach Anspruch 1, das eine Mengenbestimmungstabelle umfaßt, die Zusammensetzungen umfaßt, die gereinigtes virales HIV p24 enthalten.

8. Kit nach Anspruch 7, wobei in der Mengenbestimmungstabelle die Zusammensetzungen, die gereinigtes virales HIV p 24 enthalten, darüberhinaus einen Puffer enthalten, und wobei die Mengenbestimmungstabelle darüberhinaus eine Blindzusammensetzung umfaßt, die den Puffer enthält und in der gereinigtes HIV p 24 fehlt.

9. Kit nach Anspruch 8, wobei die Zusammensetzungen der Mengenbestimmungstabelle gefriergetrocknet sind.

10. Kit nach Anspruch 9, wobei in den Zusammensetzungen der Mengenbestimmungstabelle Tris der Puffer ist, und wobei die Zusammensetzungen darüberhinaus NaCl enthalten.

## Patentansprüche (Patentansprüche für folgende(n) Vertragsstaat(en): ES)

1. Verfahren zur quantitativen Bestimmung von HIV p24 in einer biologischen Probe, das folgende Schritte umfaßt:
In-Kontakt-Bringen der Probe mit einem Antikörper, der für HIV p24 Antigen spezifisch ist, wodurch ein nachweisbarer Antigen-Antikörper-Komplex gebildet wird;
und quantitative Bestimmung der Menge an Antigen-Antikörper-Komplex, der gebildet wurde, wobei als Standard eine Mengenbestimmungstabelle verwendet wird, die Zusammensetzungen umfaßt, die gereinigtes virales HIV p24 enthalten.

2. Verfahren nach Anspruch 1, wobei in der Mengenbestimmungstabelle die Zusammensetzungen, die gereinigtes virales HIV p 24 enthalten, Lösungen sind, die das gereinigte virale HIV p 24 und einen Puffer enthalten, und wobei die Konzentrationen des gereinigten viralen HIV p24 in den Lösungen von 1 bis 300 pg/ml variieren, und wobei die Mengenbestimmungstabelle darüberhinaus eine Blindzusammensetzung umfaßt, die eine Lösung ist, welche den Puffer enthält und in der gereinigtes HIV p 24 fehlt.

3. Verfahren nach Anspruch 1, wobei die Zusammensetzungen, die gereinigtes virales HIV p24 enthalten, darüberhinaus einen Puffer enthalten.

4. Verfahren nach Anspruch 3, wobei die Zusammensetzungen, die gereinigtes virales HIV p24 und einen Puffer enthalten, in gefriergetrockneter Form vorliegen.

5. Verfahren nach Anspruch 3 oder 4, wobei Tris der Puffer ist, und wobei die Zusammensetzungen, die gereinigtes virales HIV p24 und Tris enthalten, darüberhinaus NaCl enthalten.

## Revendications (Revendications pour l'(les) Etat(s) contractant(s) suivant(s): AT, BE, CH, DE, FR, GB, IT, LI, LU, NL)

1. Procédé de détermination quantitative de p24 de VIH dans un échantillon biologique, comprenant les étapes consistant à :
mettre ledit échantillon en contact avec un anticorps spécifique de l'antigène p24 de VIH, un complexe antigène-anticorps détectable étant formé, et
déterminer la quantité de complexe antigène-anticorps formé en utilisant comme étalon un panel de détermination quantitative qui comprend des compositions comprenant de la p24 de VIH virale purifiée.

2. Procédé selon la revendication 1, dans lequel, dans le panel de détermination quantitative, les compositions comprenant la p24 de VIH virale purifiée sont des solutions comprenant ladite p24 de VIH virale purifiée et un tampon et les concentrations de ladite p24 de VIH virale purifiée dans lesdites solutions varient entre 1 et 300 pg/ml et le panel de détermination quantitative comprend en outre une composition témoin qui est une solution qui comprend ledit tampon et est exempte de ladite p24 de VIH purifiée.

3. Composition convenant comme étalon de p24 de VIH à utiliser dans le procédé selon la revendication 1, comprenant de la p24 de VIH virale purifiée et un tampon.

4. Composition selon la revendication 3 sous forme lyophilisée.

5. Composition selon la revendication 3, dans laquelle ledit tampon est du Tris et la composition comprend en outre NaCl.

6. Composition selon la revendication 4, dans laquelle ledit tampon est du Tris et la composition comprend en outre NaCl.

7. Kit à utiliser pour la détermination quantitative de p24 de VIH dans un échantillon biologique selon le procédé de la revendication 1, comprenant :
un panel de détermination quantitative qui comprend des compositions comprenant de la p24 de VIH virale purifiée.

8. Kit selon la revendication 7, dans lequel, dans le panel de détermination quantitative, les compositions comprenant de la p24 de VIH virale purifiée comprennent également un tampon et le panel de détermination quantitative comprend en outre une composition témoin qui comprend ledit tampon et est exempte de ladite p24 de VIH purifiée.

9. Kit selon la revendication 8, dans lequel les compositions du panel de détermination quantitative sont lyophilisées.

10. Kit selon la revendication 9, dans lequel, dans les compositions du panel de détermination quantitative, ledit tampon est du Tris et les compositions comprennent en outre NaCl.

## Revendications (Revendications pour l'(les) Etat(s) contractant(s) suivant(s): ES)

1. Procédé de détermination quantitative de p24 de VIH dans un échantillon biologique, comprenant les étapes consistant à :
mettre ledit échantillon en contact avec un anticorps spécifique de l'antigène p24 de VIH, un complexe antigène-anticorps détectable étant formé, et
déterminer la quantité de complexe antigène-anticorps formé en utilisant comme étalon un panel de détermination quantitative qui comprend des compositions comprenant de la p24 de VIH virale purifiée.

2. Procédé selon la revendication 1, dans lequel, dans le panel de détermination quantitative, les compositions comprenant de la p24 de VIH virale purifiée sont des solutions comprenant ladite p24 de VIH virale purifiée et un tampon et les concentrations de ladite p24 de VIH virale purifiée dans lesdites solutions varient entre 1 et 300 pg/ml et le panel de détermination quantitative comprend en outre une composition témoin qui est une solution qui comprend ledit tampon et est exempte de ladite p24 de VIH purifiée.

3. Procédé selon la revendication 1, dans lequel lesdites compositions comprenant la p24 de VIH virale purifiée comprennent en outre un tampon.

4. Procédé selon la revendication 3, dans lequel lesdites compositions comprenant de la p24 de VIH virale purifiée et un tampon sont sous une forme lyophilisée.

5. Procédé selon la revendication 3 ou 4, dans lequel ledit tampon est du Tris et lesdites compositions comprenant de la p24 de VIH virale purifiée et le Tris comprennent en outre NaCl.
